# EUROPEAN PATENT SPECIFICATION

(11) **EP 0 493 737 B1**
(45) Date of publication and mention of the grant of the patent: **24.07.2002**
(21) Application number: 91121597.8
(22) Date of filing: 17.12.1991
(51) Int. Cl.: A61K 38/18, C07K 14/50

(54) **Agent for the treatment of bone diseases containing basic fibroblast growth factor**
Einen basischen Fibroblastwachstumsfaktor enthaltende Mittel zur Behandlung von Knochenkrankheiten
Agent pour le traitement des maladies osseuses contenant du facteur basique de croissance des fibroblastes

(30) Priority: 19.12.1990 JP 41916890; 28.05.1991 JP 15251791
(43) Date of publication of application: 08.07.1992
(73) Proprietor: KAKEN PHARMACEUTICAL CO., LTD., Tokyo 113 (JP)
(72) Inventor: Hanada, Keigo, c/o Kyoto Research Institute, Shinomiya, Yamashina-ku, Kyoto 607 (JP); Hiyama, Yoshiyuki, c/o Kyoto Research Institute, Shinomiya, Yamashina-ku, Kyoto 607 (JP); Tamura, Makoto, c/o Kyoto Research Institute, Shinomiya, Yamashina-ku, Kyoto 607 (JP)
(74) Representative: Gille Hrabal Struck Neidlein Prop Roos

(56) References cited:
- EP-A- 0 320 148
- WO-A-87/01728
- WO-A-89/04832
- WO-A-90/02800
- WO-A-90/05184
- FR-A- 2 642 086
- ENDOCRINE REVIEWS vol. 8, no. 2, 1987, pages 95-114, US; D. GOSPODAROWICZ et al.: "Structural characterization and biological functions of fibroblast growth factor"
- BIOLOGICAL ABSTRACT no. 89055692; P. ASPENBERG et al.: "Fibroblast growth factor stimulates bone formation. Bone induction studied in rats." & Acta Orthopaedica Scandinavica 1989, vol. 60, no. 4, pages 473-476

## Description

### BACKGROUND OF THE INVENTION

### FIELD OF THE INVENTION

The present invention relates to the use of analogues of human fibroblast growth factor for the treatment of bone diseases such as traumatic fracture, fatigue fracture, pathologic fracture, etc.

### STATEMENT OF THE PRIOR ART

For the treatment of traumatic fracture or fatigue fracture, a method consists of reposing and fixing an injured site and then leaving to a natural healing-ability of the patient himself. In natural healing of fractured bone, bone union requires a number of days, e.g., about 2 weeks for metacarpal bone, about 5 weeks for forearm bone and about 12 weeks for neck of femur, in average. In this case, pharmacological treatment is not usually performed but nutritional treatment such as an administration of calcium, is done, if necessary and desired.

Further in pathologic fracture resulted from osteoporosis, diabetes, etc., natural healing-ability of a patient is lowered and hence, in addition to the reposition and fixing of an injured site, pharmacological treatment, an administration of estrogen or calcitonin, etc., is applied; or the above pharmacological treatment is performed in combination with the nutritional treatment. The pharmacological treatments or those in combination with nutritional treatment are also applied to the treatment of reduced bone strength resulted from diseases such as osteoporosis or diabetes.

Under the actual situation that fracture treatment has been left to the natural healing-ability of the patient himself, development of an oral agent for accelerating fracture healing comprising 24,25-dihydroxycholecalciferol was recently reported: the agent is usable as a drug for shortening a period of fracture healing (Japanese Patent Application KOKAI No. 63-310828). Reports have been also made on agents for the treatment of bone diseases such as an agent for accelerating bone formation, comprising rentinan (Japanese Patent Application KOKAI No. 63-17828), an agent for the treatment of bone diseases comprising at least one selected from the group consisting of chitin, chitosan and derivatives thereof (Japanese Patent Application KOKAI No. 63-156726), as new drugs for shortening a period for fracture healing or improving reduced bone strength accompanied by various diseases; or an agent comprising physiologically active ganglioside of retina, and the like (Japanese Patent Application KOHYO No. 63-502035).

The basic fibroblast growth factor (hereinafter abbreviated as bFGF) is a peptidic cell growth factor which was confirmed to be present in pituitary, brain, retina, corpus luteum, adrenal, kidney, placenta, prostate and thymus. It is known that bFGF includes the proliferation of mesodermal cells including vascular endothelial cells ["Cell Growth Factor Part II", edited by Tissue Culture Association, Japan, pages 15 to 20, published by Asakura Publishing Co.). In recent years, bFGF is also isolated from cartilage and bone [J.B.C., 260, 2399-2403 (1985); ibid., 261, 12665-12674 (1986)].

It was expected that the bFGF would effectively accelerate the fracture healing, since bFGF evokes a proliferation of mesodermal cells including vascular endothelial cells and is present in cartilage or bone. The in vivo test on bone formation reveals that bFGF accelerates bone formation only in combination with decalcified bovine bone containing a bone growth factor [Act. Orthop. Scand., 60, 473-476 (1989)]. bFGF has a high structural similarity to an acidic fibroblast growth factor which accelerated callus formation in fractured ends at the early stage of fracture healing but inhibited the matrix synthesis of cartilage or bone and was thus evaluated to be inferior to control group, in bone strength at the site of callus formation [J. Orthopaedic Res., 8, 364-371 (1990)]. bFGF is also recognized to have a high similarity to the acidic fibroblast growth factor in view of the action in vivo.

In the trend of the studies on bFGF as described above, the present inventors have shown for the first time that an analogue bFGF alone, can accelerate callus formation at a fracture site, accelerate bone formation for bone defect, promote bone union, improve the strength at a fracture site and increase the bone strength.

### SUMMARY OF THE INVENTION

The present invention has been made in view of the actual situation of aforementioned treatment for bone diseases, and an object of the present invention is to provide a agent for the treatment of bone diseases which can shorten a healing period of various fractures including acceleration of bone formation for bone defect, improve bone strength of fused bones and also improve reduced bone strength accompanied by various diseases.

As a result of extensive investigation to accomplish the aforesaid object, the present inventors have succeeded in developing useful drugs as agents for the treatment of bone diseases using a basic fibroblast growth factor (bFGF) and have come to complete the present invention.

That is, the agent for the treatment of bone diseases used according to the present invention is characterized by comprising as an effective ingredient a basic fibroblast growth factor (bFGF) analogue according to claim 1.

### BRIEF DESCRIPTION OF THE DRAWINGS

Fig. 1 shows a soft X-ray photograph (x 2.3 times) of fractured bone (left fibula) in Group B of Example 1 three weeks after fracture in Example 1.
Fig. 2 shows a soft X ray photograph (x 2.3 times) of fractured bone (left fibula) in the control group three weeks after fracture in Example 1.
Fig. 3 shows a soft X ray photograph (x 2.3 times) of fractured bone (left fibula) in Group B of Example 3 three weeks after fracture in Example 3.
Fig. 4 shows a soft X ray photograph (x 2.3 times) of fractured bone (left fibula) in Control Group B three weeks after fracture in Example 3.
Fig. 5 shows a microscopic photograph (x 32, taken from the subject showing conspicuous bone formation) of a sliced tissue section of the defect (right tibila) in Group of Example 4 one week after bone defect in Example 4.
Fig. 6 shows a microscopic photograph(x 32, taken from the subject showing slight bone formation) of a sliced tissue section of the defect (right tibila) in Control Group 4B one week after bone defect in Example 4.

### DETAILED DESCRIPTION OF THE PREFERRED EMBODIMENTS

Hereinafter the present invention is described in detail.

bFGF as an effective ingredient of a medicament for the treatment of bone diseases is a well known growth factor as described above and its presence is confirmed in human, bovine, mouse, rat, etc. Basically, bFGF from any animal origin has the same activity in vivo. However, where agents for the treatment of bone diseases are applied to humans, it is particularly preferred to use a bFGF having the same amino acid sequence as that of bFGF produced in human body (hereinafter referred to as human bFGF), in view of antigenicity.

In the agent used according to the present invention for the treatment of bone diseases, the following analogue of bFGF is used: a human basic fibroblast growth factor (bFGF) analogue according to claim 1.

In order to supply an agent for the treatment of bone diseases stably in an industrial scale, it is in general particularly preferred to produce bFGF or its analogues in a microorganism such as E. coli, etc. or in a cultured cell by genetic recombination technology, since bFGF is present in vivo in an extremely low trace amount.

The use of the present invention for the treatment of bone diseases comprises as an effective ingredient the bFGF analogues described above. Specific forms of the agent include a solution comprising bFGF and/or its analogue, physiological saline or other conventional auxiliary agents (glucose, sucrose, buffer, etc.), an injection or a spray using the solution, a gel, an ointment, etc. The agent of the present invention for the treatment of bone diseases may take a route of topical application or general application, but topical application is particularly preferred.

The use according to the present invention is related with A through D described below.
A. Various traumatic fractures
B. Various fatigue fractures
C. Pathologic fractures
   a. Fracture accompanied by osteoporosis primary osteoporosis (senile, postmenopausal, juvenile) secondary osteoporosis {(hyperthyroidism, Cushing's syndrome (caused by steroid administration), acromegalia, hypogonadism [hypopituitarism, Klinfelter's syndrome, Turner's syndrome], osteogenesis imperfecta, hypophosphatasia, homocystinuria, immobilization osteoporosis, diabetes}
   b. Fracture accompanied by osteomalacia
   c. Fracture accompanied by malignant tumor
   d. Fracture accompanied by multiple myeloma
   e. Fracture accompanied by osteogenesis inperfect acongenita
   f. Fracture accompanied by osteocystoma
   g. Fracture accompanied by suppurative osteomyelitis
   h. Fracture accompanied by osteopetrosis
   i. Fracture accompanied by nutrition disorder .
D. Bone fracture in patients having diabetes or any disease treated by steroid administration.

An effective dose of the agent for the treatment of bone diseases in accordance with the present invention varies depending on kind of application, degree of bone diseases, age or condition of the patient, etc. but is generally in the range of about 0.1 µg to 10 mg/fracture site, as the effective ingredient, in the case of fracture. Generally for the purpose of accelerating healing, a particularly preferred route for application is to administer the agent in direct contact with the fracture site. However, the agent may also be subcutaneously applicable or directly injectable around the fracture site.

The agent for the treatment of bone diseases in accordance with the present invention may be also applied to the treatment of bone diseases of domestic animal, pet mammal, raised wild animal, etc. In this case, for example, bovine bFGF and/or its analogue may be used. The same preparation forms and effective dose as in the case of human may apply.

Hereinafter the present invention is described with reference to the examples.

### Example 1

Five (5) to six (6) SD strain male rats (weighing 300 to 325 g) of 10 weeks age were grouped in one group, and 9 groups in total were prepared. Sodium pentobarbital solution (concentration of 50 mg/ml, trademark: NENBUTAL, manufactured by Dainippon Pharmaceutical Co., Ltd.) was intraperitoneally administered to each animal in a dose of 0.20 ml to anesthesize. Then, the hair at the left lower limb was cut and the skin and the muscular layer were incised in a length of 1 cm to expose the fibula. The fibula was cut out at the center, using bone scissors.

The first 3 groups were used as control groups. After the cut fibula was reposed with tweezers, 36 µl of 0.1 M phosphate buffered saline was dropped onto the reposed site and the incised skin was sutured. After diluted iodine tincture was applied to the wound after operation, 0.1 ml of 10-fold physiological saline dilution of sodium penicillin G-streptomycin sulfate solution [penicillin concentration of 10000 U/ml, streptomycin concentration of 10000 µg/ml, manufactured by Gibco Co.] was subcutaneously administered.

The remaining 6 groups were used as the groups treated according to the invention. In 3 groups (hereinafter referred to as Group A of Example 1) out of the 6 groups, 36 µl of a human bFGF solution (concentration: 0.5 µg/ml) was dropwise applied to each animal, instead of 36 µl of 0.1 M phosphate-buffered saline. Said human bFGF solution was previously prepared by diluting with 0.1 M phosphate-buffered saline, a solution (concentration of 3.3 mg/ml) of human bFGF (polypeptide shown by sequence No. 3 in Table 3 attached; hereinafter human bFGF refers to this polypeptide throughout the specification) expressed in E. coli, which had been obtained by genetic recombination technology. Thereafter the groups were otherwise treated in a manner similar to the control groups.

In the remaining 3 groups of this invention (hereinafter referred to as Group B of Example 1), 36 µl of a human bFGF solution (concentration: 50 µg/ml) obtained by diluting a solution (concentration: 3.3 mg/ml) of human bFGF with 0.1 M phosphate-buffered saline was dropwise applied to each animal, instead of 36 µl of the human bFGF solution (concentration: 0.5 µg/ml) used in Group A of Example 1, and the groups were treated otherwise in a manner similar to Group A of Example 1.

Respectively one group was chosen from the control groups (3 groups in total), Group A of Example 1 (3 groups in total) and Group B of Example 1 (3 groups in total) every 1, 2 and 3 other weeks after fracture. Each animal in the groups chosen was anesthesized with ether and bled from the carotid artery to death. The right and left leg bones were withdrawn and immediately immersed in 10% neutral formalin buffer to fix them.

Using these fixed specimens, the following items were determined.

### (a) Determination of cross sectional area of fractured callus

After fixing with 10% neutral formalin buffer, photographs of the leg bones (tibia and fibula) were taken by soft X rays (trademark of equipment used: SOFTEX-CMB, manufactured by Japan Softex Co., Ltd., conditions: 24-30 kV, 1-3 mA) to prepare soft X ray pictures. From the pictures, photographs enlarged to 10 times with a stereoscopic microscope were prepared. Based on these photographs, the cross sectional area of callus was determined using an image analysis device [trademark: Oscondigitizer Model SQ-3100F, manufactured by PHOTORON Co.].

### (b) Determination of callus volume

After fixing with 10% neutral formalin buffer, the volume of the left and right fibulae (left: fractured bone, right: normal bone) was determined, respectively, with a device for measuring volume [trademark, PLETHYSMOMETER, manufactured by Ugo Basile Co., Ltd.]. A value obtained by subtracting the measurement data of the right fibula from that of the left fibula was determined to be the volume of callus.

### (c) Determination of bone mineral content (BMC) and bone mineral density (BMD) of fractured callus

After fixing with 10% neutral formalin buffer, BMC of the left and right fibulae (left: fractured bone, right: normal bone) was determined, respectively, with a device for measuring BMC [trademark, Model DCS-600, manufactured by Aloka Co., Ltd.]. A value obtained by subtracting the measurement data of the right fibula from that of the left fibula was determined to be the BMC of callus. Furthermore, BMD of the left and right fibulae were determined, respectively, using the same device and BMD was determined in a manner similar to the case of the BMC of callus.

### (d) Biomechanical analyses for fractured fibula

After the left and right fibulae were withdrawn 3 weeks after fracture and fixed in 10% neutral formalin buffer, break energy in the specimens thus obtained was determined, with a device for measuring bone metabolism [trademark, RHEOROMETER MAX, manufactured by Iio Electric Co., Ltd.]. Furthermore, a ratio (percentage) in bone strength of the left fibula (fractured bone) to that of the right fibula (normal bone) was determined as an increment of bone strength.

### (e) Histological examination

After fixing in 10% neutral formalin buffer for a week, the left and right fibulae were decalcified with 10% EDTA (ethylenediamine tetraacetate) solution for 10 days, respectively. After decalcification, the bones were washed with water for 12 hours and embedded in paraffin in a conventional manner, from which sliced tissue sections each having a thickness of 1 to 4 µm were prepared by a microtome, respectively. Then, each specimen was stained with hematoxylin-eosine. The stained specimens were microscopically examined and pictures of them were taken.

From these results, the results of measurements of cross sectional area, volume, BMC, and BMD of callus and the results of test on bone strength of the fractured bones are shown in Table 1.

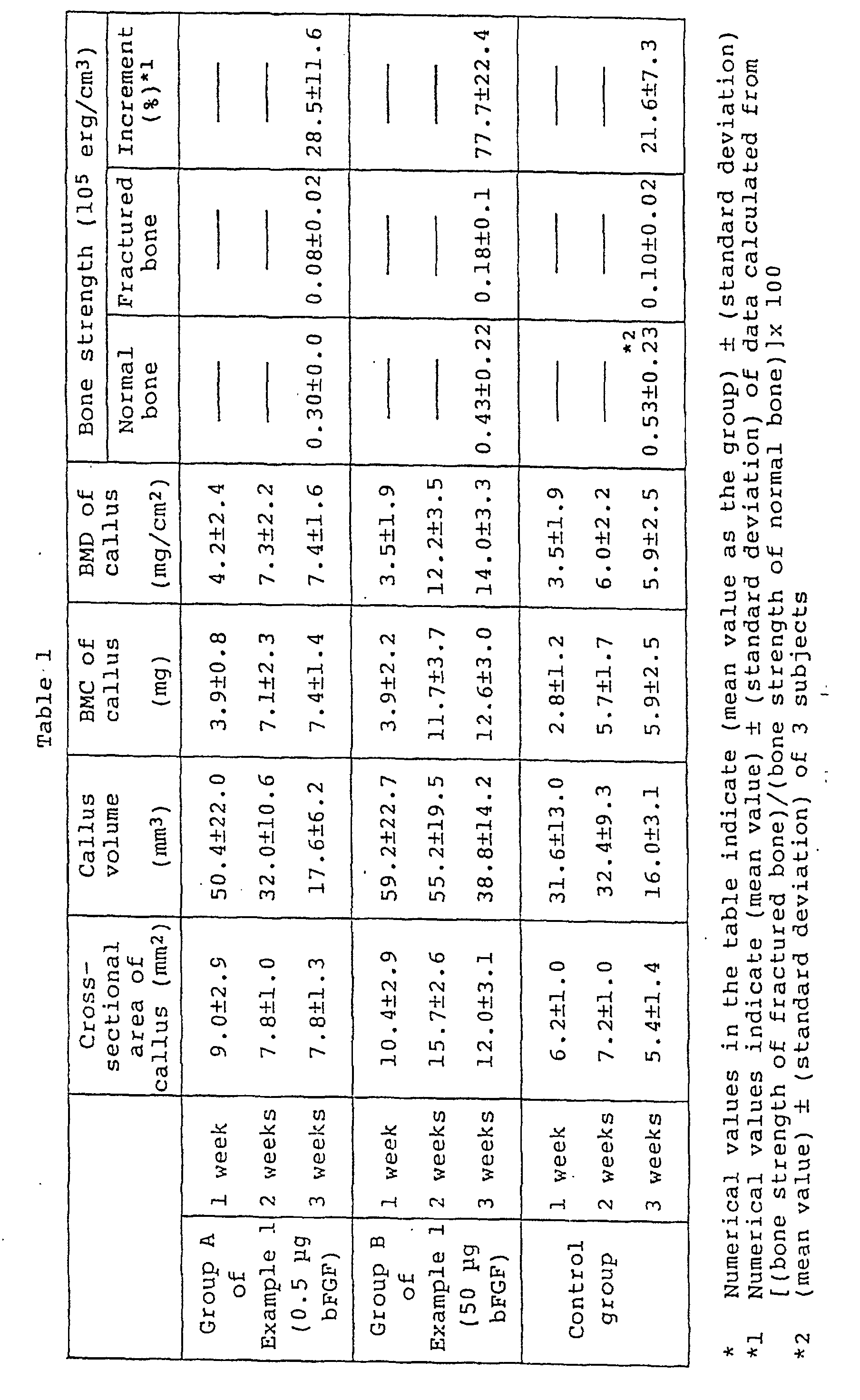

As is evident from Table 1, the cross sectional area of callus and callus volume one week after fracture increased in Group A of Example 1 and Group B of Example 1, as compared to the cross sectional area of callus and callus volume in the control groups. On and after one week, both the cross sectional area of callus and callus volume gradually decreased both in the groups of this invention and in the control groups, but the cross sectional area of callus and callus volume were larger in the groups of this invention even 3 weeks after. These results reveal that the agent used according to the present invention for the treatment of bone diseases accelerates the callus formation.

Further with respect to BMC and BMD of fractured callus, no remarkable difference was noted between the groups of the present invention and the control groups one week after fracture. In the groups of the present invention, however, both BMC and BMD increased to about 2.3 times that of the control groups. These results reveal that the agent for the treatment of bone diseases has an excellent effect on fracture healing. This is also evident from the fact that in Groups A and B of Example 1, the increment in the test on bone strength after 3 weeks is higher than in the control groups.

Furthermore, the above mentioned higher increment in the test on bone strength indicates that the inventive use for the treatment of bone diseases is excellent in improving the bone strength or the strength at the fracture site of bone.

The results stated above are clear also from the soft X-ray photographs, some of which are attached as the drawings.

In the soft X-ray photograph in Group B of Example 1 taken one week after fracture, a large callus image is noted and the formation of new bone having weak transmittance is recognized beneath the periosteum around the fractured site of fibula. Turning to a soft X-ray photograph in the control group taken one week after fracture, the formation of callus having strong transmission is noted but its area is smaller than in Group B of Example 1.

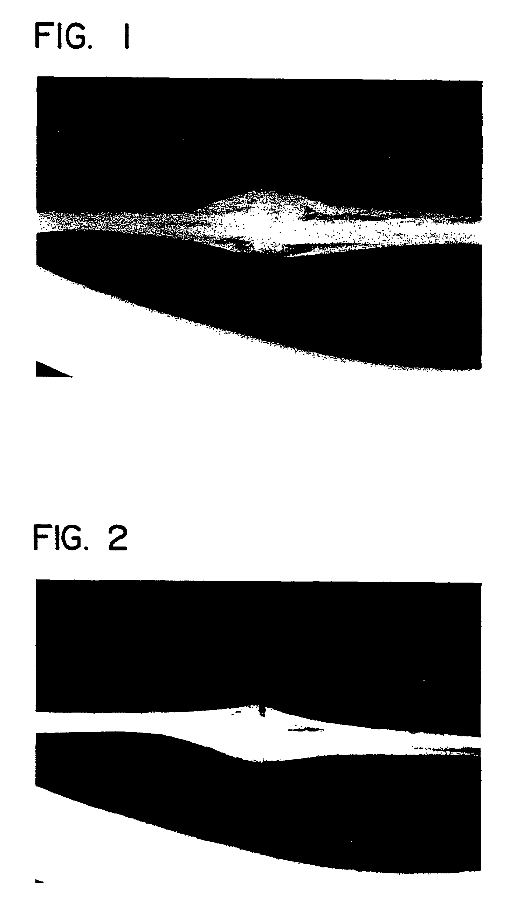

Fig. 1 shows a soft X-ray photograph in Group B of Example 1 taken 3 weeks after fracture. In the photograph, the transmission of callus decreases indicating that new bone formation is markedly accelerated. Turning to Fig. 2 which shows a soft X-ray photograph of the control group taken 3 weeks after fracture, decreased transmission of callus means a formation of new bone but its degree is smaller than in Group B of Example 1.

It is also evident from the results of histopathological survey that the agent for the treatment of bone diseases promotes callus formation and exhibits an excellent effect on bone formation.

That is, as is clear from a microscopic photograph (x 5 times) of the sliced tissue sections, proliferation of cartilage, proliferation of osteogenic mesenchymal cells derived from periosteum, etc., and formation of fibrous new bone around fractured site are remarkable. On the other hand, as is clear from a microscopic photograph in the control group one week after fracture, proliferation of cartilage, proliferation of osteogenic mesenchymal cells, and formation of fibrous new bone around fractured site are also noted in the control group one week after fracture but their degrees are smaller than in Group B of Example 1.

Further as is clearly seen from a microscopic photograph in Group B of Example 1 three weeks after fracture, osteogenic mesenchymal cells showed marked proliferation within an enlarged callus and the fracture ends are united by a number of newly formed fibrous bones but cartilage slightly remains around fracture site. Formation of marrow cavity is also noted between the fibrous new bones. On the other hand, as is clear from a microscopic photograph in the control group 3 weeks after fracture, callus itself is small and formation of cartilage is poor in the control group 3 weeks after fracture. In addition, the fracture ends are still separated by cartilage.

### Example 2

Firstly, five (5) to six (6) SD strain male rats (weighing 300 to 325 g) of 10 weeks age were grouped in one group and 6 groups in total were prepared. In the same manners as in Example 1, sodium pentobarbital solution (concentration of 50 mg/ml, trademark: NENBUTAL, manufactured by Dainippon Pharmaceutical Co., Ltd.) was intraperitoneally administered to each animal in a dose of 0.20 ml to anesthesize. Then, the hair at the left lower limb was cut and the skin and the muscular layer were incised in a length of 1 cm, respectively, to expose the fibula. The fibula was cut out at the center using bone scissors.

Two groups out of the 6 groups were used as control groups and treated in a manner similar to the control groups in Example 1.

The remaining 4 groups were used as the groups of this invention. In 2 groups (hereinafter referred to as Group A of Example 2) out of the 4 groups, 36 µl of a human bFGF solution (concentration: 1 µg/ml) obtained by diluting with o.1 M phosphate-buffered saline, a solution (concentration: 3.3 mg/ml) of human bFGF prepared in a similar manner to Example 1 was dropwise applied to each animal, instead of 36 µl of the human bFGF solution (concentration: 0.5 µg/ml) used in Group A of Example 1, and the groups were treated otherwise in a manner similar to Group A of Example 1.

In the remaining 2 groups of this invention (hereinafter referred to as Group B of Example 2), 36 µl of a human bFGF solution (concentration: 100 µg/ml) obtained by diluting with 0.1 M phosphate buffered saline, a solution (concentration: 33 mg/ml) of human bFGF prepared in a similar manner to Example 1 was dropwise applied to each animal, instead of 36 µl of the human bFGF solution (concentration: 1 µg/ml) used in Group A of Example 2, and the groups were treated otherwise in a manner similar to Group A of Example 2.

One group was chosen from the control groups (2 groups in total), Group A of Example 2 (2 groups in total) and Group B of Example 2 (2 groups in total) every 1 and 3 other weeks after fracture. The right and left leg bones were treated in a manner similar to Example 1 to prepare specimens fixed in 10% neutral formalin buffer.

Then, the cross sectional area of callus, callus volume, BMC and BMD of callus were determined in a manner similar to Example 1. These measurement results are shown in Table 2.

**Table 2**

| | Cross sectional area of callus (mm²) | Callus volume (mm³) | BMC of callus (mg) | BMD of callus (mg/cm²) |
|---|---|---|---|---|
| Group A of Example 2 (1 µg of bFGF) : | | | | |
| 1 week | 9.5±2.6 | 58.5±22.9 | 4.3±0.5 | 3.9±0.4 |
| 3 weeks | 7.9±1.7 | 26.5±7.0 | 4.6±2.2 | 6.4±3.0 |

| Group B of Example 2 (100 µg of bFGF): | | | | |
|---|---|---|---|---|
| 1 week | 10.5±1.3 | 49.5±7.5 | 2.5±1.5 | 3.0±2.0 |
| 3 weeks | 8.7±1.5 | 24.5±7.5 | 9.4±1.3 | 9.6±2.1 |

| Control group: | | | | |
|---|---|---|---|---|
| 1 week | 6.7±1.2 | 32.0±4.0 | 0.6±0.5 | 1.4±0.9 |
| 3 weeks | 5.6±0.7 | 13.6±8.4 | 3.0±1.6 | 3.3±1.8 |
| *: Numerical values in the table indicate: (mean value in the group) ± (standard deviation) | | | | |

As is evident from Table 2, the measurement results in Example 2 have the same tendency as those in Example 1, indicating that the agent for the treatment of bone diseases accelerates callus formation and has an excellent effect on fracture healing.

### Example 3

Firstly, many male rats of SD strain (weighing 300 to 325 g) of 10 weeks age were prepared and a solution of streptozotocin (concentration of 40 mg/ml) in sodium citrate buffer (pH = 4.9) was intravenously administered through the tail vein in a dose of 0.1 ml/100 g of body weight.

Then, one week after the administration of streptozotocin, blood sugar level of each animal was determined by the method for measuring absorbance using a blood sugar measurement kit (trademark: NEW BLOOD SUGAR TEST, manufactured by Boehringer Yamanouchi Co., Ltd.). Rats showing a blood sugar level of 250 mg/dl or more were chosen as model rats for diabetes. Five (5) or six (6) rats were divided into one group and 8 groups in total were prepared.

Further five (5) or six (6) SD strain male rats (weighing 300 to 325 g) of 10 weeks age were grouped in one group, and 2 groups in total were prepared for non-diabetes control group.

Then the left fibula was cut out at the center using bone scissors in a manner similar to Example 1.

Among 8 groups of model rats for diabetes, 4 groups were made control groups. With respect to each animal in 2 groups (hereinafter referred to as Control Group A) out of these 4 groups, the cut fibula was reposed with tweezers followed by suture of the incised skin, application of diluted iodine tincture to the wound in operation and subcutaneous administration of 10-fold dilution of sodium penicillin G-streptomycin sulfate with physiological saline to the sutured site in a manner similar to Example 1.

In the remaining 2 groups (hereafter referred to as Control Group B) in the control groups, 36 µl of the gel of 0.1 M phosphate buffer (pH 6.5) containing fibrinogen, aprotinin and thrombin in concentrations of 41.6 mg/ml, 0.41 mg/ml and 33.3 U/ml, respectively, were dropwise applied to each animal, instead of 36 µl of 0.1 M phosphate-buffered saline in the control group of Example 1. The groups were treated otherwise in a manner similar to the control group of Example 1.

The remaining 4 groups of model animals for diabetes were made the groups of this invention. In 2 groups (hereinafter referred to as Group A of Example 3) out of the 4 groups, 36 µl of the gel further containing 0.5 µg/ml of human bFGF was dropwise applied to each animal, instead of 36 µl of the gel in Control Group B. The animal was treated otherwise in a manner similar to Control Group B.

In the remaining 2 groups of this invention (hereafter referred to as Group B of Example 3), 36 µl of the gel further containing 50 µg/ml of human bFGF was dropwise applied to each animal, instead of 36 µl of the gel in Control Group B. The animal was treated otherwise in a manner similar to Control Group B.

Each one group was chosen from Control Group A (2 groups in total), Control Group B (2 groups in total), Group A of Example 3 (2 groups in total), Group B of Example 3 (2 groups in total) and non-diabetes control groups (2 groups in total) every 1 and 3 other weeks after fracture. The right and left leg bones were treated in a manner similar to Example 1 to prepare specimens fixed in 10% neutral formalin buffer.

Then, volume, BMC and BMD of fracture callus were determined in a manner similar to Example 1. Furthermore, histopathological examination was performed. In these results, the measurement data on callus volume, BMC and BMD of callus are shown in Table 3.

**Table 3**

| | Callus volume (mm³) | BMC of callus (mg) | BMD of callus (mg/cm²) |
|---|---|---|---|
| Group A of Example 3 (0.5 µg of bFGF): | | | |
| 1 week | 27.2±11.5 | 2.3±1.0 | 1.9±1.0 |
| 3 weeks | 6.5±3.0 | 2.6±0.2 | 2.8±1.3 |

| Group B of Example 3 (50 µg of bFGF): | | | |
|---|---|---|---|
| 1 week | 51.6±8.1 | 4.2±1.5 | 3.9±1.2 |
| 3 weeks | 26.0±5.8 | 7.8±2.0 | 11.3±1.7 |

| Control group A: | | | |
|---|---|---|---|
| 1 week | 17.2±4.6 | 0.2±0.1 | 0.4±0.2 |
| 3 weeks | (6,10)*¹ | (1.3,1.2)*¹ | (3.1,1.7)*¹ |

| Control group B: | | | |
|---|---|---|---|
| 1 week | 15.5±8.0 | 2.6±0.1 | 2.7±0.5 |
| 3 weeks | 6.0±2.0 | 1.7±0.5 | 2.5±0.9 |

| Non-diabetes control group: | | | |
|---|---|---|---|
| 1 week | 26.3±7.2 | 2.2±1.4 | 1.9±0.8 |
| 3 weeks | 11.3±3.5 | 4.2±2.2 | 4.6±2.2 |

| | | | |
|---|---|---|---|
| *: Numerical values in the table indicate: (mean value in the group) ± (standard deviation) | | | |
| *1: Each measurement value of the two animals | | | |

As is evident from Table 3, the measurement results in Example 3 have the same tendency as those in Example 1 and Example 2. The results reveal that the treatment of bone diseases accelerates callus formation and excellent bone formation effect also in model rats for diabetes. Therefore, the use of the present invention for the treatment of bone diseases has the effect or accelerating callus formation and leads to an excellent bone formation effect also in pathologic fracture.

These effects are evident also from soft X-ray photographs, some of which are attached as the drawings.

In a soft X-ray photograph in Group B of Example 3 one week after fracture, a large callus image is noted, whereas the callus image noted in a soft X-ray photograph in Control Group B one week after fracture is small and formation of callus is very poor.

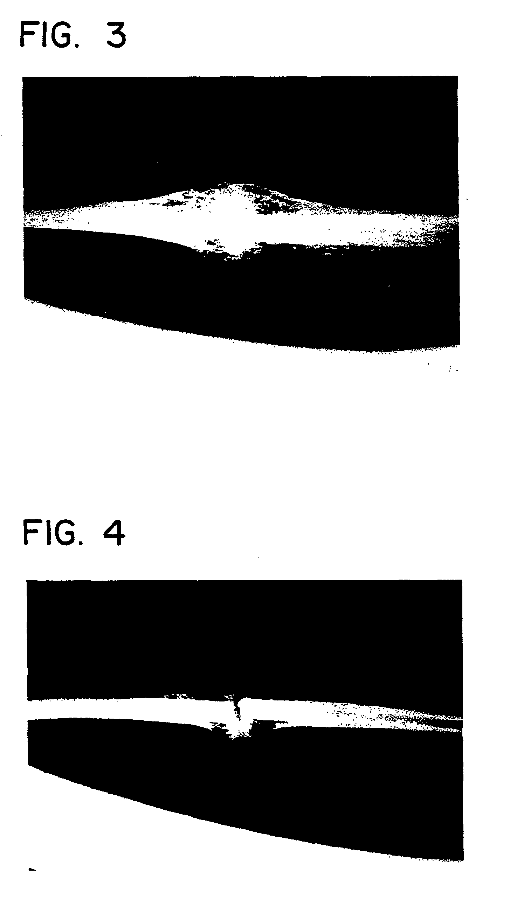

Further in a soft X-ray photograph (3 weeks after fracture) of Group B of Example 3 which is attached as Fig. 3, formation of many bones is noted in a large callus, indicating the sign that union of the fracture site has started. To the contrary, no marked enlargement of callus is noted in a soft X-ray photograph (3 weeks after fracture) in Control Group B which is attached as Fig. 4. Union at the fracture site is not noted, either.

It is evident also from histopathological examination that the use of the present invention for the treatment of bone diseases promotes callus formation and has an excellent effect on fracture healing also in diabetic animal model.

That is, as is clearly seen from the microscopic photograph (x 5 times) of sliced tissue sections, callus formation, cartilage formation and proliferation of osteogenic mesenchymal cells are remarkable in Group B of Example 3 one week after fracture. To the contrary, in a microscopic photograph of sliced tissue sections in Control Group B taken one week after fracture, callus formation, cartilage formations, and proliferation of osteogenic mesenchymal cells are also noted in Control Group B one week after fracture but cartilage and osteogenic mesenchymal cells are poorer than in Group B of Example 3.

Further as is clear from the microscopic photograph of sliced tissue section, the fracture ends are still separated by cartilage or mesenchymal cells in Group B of Example 3 three weeks after fracture, but formation of new fibrous bones is remarkable and the fracture site has begun to be united from the periphery of callus. To the contrary, as is clear from a microscopic photograph of sliced tissue section, in Control Group B taken three weeks after fracture, the fracture site is still separated by the connecting tissue including osteogenic mesenchymal cells, and formation of cartilage and fibrous new bones is poor.

### Example 4

Firstly five (5) SD strain male rats (weighing 300 to 325 g) of 10 weeks age were grouped in one group, and two groups were prepared. Prednisolone was subcutaneously administered to each animal of these groups twice every other day in a dose of 25 mg/kg (as 0.5% carboxymethyl cellulose aqueous solution). These groups are hereinafter referred to as steroid-administered rat groups. On the day following the second administration, each animal was operated as described below to prepare model rat with bone defect.

Furthermore, five (5) SD strain male rats (one group, weighing 300 to 325 g) of 10 weeks age were separately prepared. The 0.5% carboxymethyl cellulose aqueous solution was subcutaneously administered to each animal of these groups twice every other day instead of prednisolone described above. This group is hereinafter referred to as non-steroid-administered rat group. On the day following the second administration, each animal was operated as described below to prepare model rat with bone defect.

### Operation (model rat with bone defect)

After each animal was anesthesized in a manner similar to Example 1, the hair at the right lower limb was cut and the skin and the muscular layer were incised in a length of 1 cm to expose the front of the tibia. Then a hole having a diameter of 1.6 mm which reached the bone marrow was made using a dental drill, at the site of 1 cm from the tibial condyle of each animal, to prepare model animal with bone defect.

After the operation, 2 µl of the gel prepared from fibrinogen, aprotinin and thrombin prepared in a manner similar to Example 3 was dropwise applied to the bone defect site (hole made by a dental drill; hereinafter the same) of each animal in the non-steroid-administered rat group (hereinafter referred to as Control Group 4A) and in one steroid-administered rat group (hereinafter referred to as Control Group 4B) of each animal. After the application, the incised skin was sutured and diluted iodine tincture was applied to the wound after operation. Then a solution of penicillin G-streptomycin sulfate solution was subcutaneously given to the sutured site in a manner similar to Example 1.

In the remaining one steroid-administered rat group (hereafter referred to as Group of Example 4), 2 µl of the gel further containing 2 µg of human bFGF was dropwise applied (dose of human bFGF: 2 µg/bone defect site) to the bone defect site of each animal, instead of the gel applied to Control Group 4A. After the application, each animal was treated as in Control Group 4A.

After the operation, prednisolone was subcutaneously administered to each animal in Group of Example 4 and in Control Group 4B in the dose described above, three times in total on the next day and Days 3 and 5 after the operation. In Control Group 4A, 0.5% carboxymethyl cellulose aqueous solution was subcutaneously administered in the dose described above to each animal three times in total on the next day and Days 3 and 5 after the operation.

One week after the operation, a specimen of the right tibila from each animal which had been fixed in 10% neutral formalin buffer was prepared in a manner similar to Example 1. After a sliced tissue section was prepared in a manner similar to Example 1, each specimen was subjected to Masson's trichrome staining. The specimen was microscopically examined histopathologically and photographed. The results of histopathological examination is shown in Table 4.

**Table 4**

| | Degree of bone formation* (number of animals) | | |
|---|---|---|---|
| | - or + | ++ | +++ |
| Group of Example 4 | 0 | 3 | 2 |
| Control group 4A | 0 | 1 | 4 |
| Control group 4B | 4 | 1 | 0 |

| | | | |
|---|---|---|---|
| * Symbols in the degree of bone formation denote the following: - : no bone formation + : slight bone formation ++ : moderate bone formation +++ : remarkable bone formation | | | |

As is clear from Table 4, moderate or remarkable bone formation was noted at the bone defect site of each animal in Group of Example 4, namely, the steroid-treated rat group administered with human bFGF. To the contrary, bone formation was hardly noted at the bone defect site in many rats in Control Group 4B, namely the steroid-treated rat group to which no human bFGF had been administered. On the other hand, natural healing proceeded normally in Control Group 4A which was the non-steroid-treated rat group and bone formation was markedly noted at the bone defect site in many rats. These results reveal that the use of the present invention for the treatment of bone diseases has an excellent effect of accelerating bone formation also at the bone defect site of rat treated with steroid (corresponding to model animal with dysfunction of bone formation).

The above results are noted more clearly by comparing microscopic photographs (x 10 times) of sliced tissue sections in the respective groups, some of which are attached as the drawings.

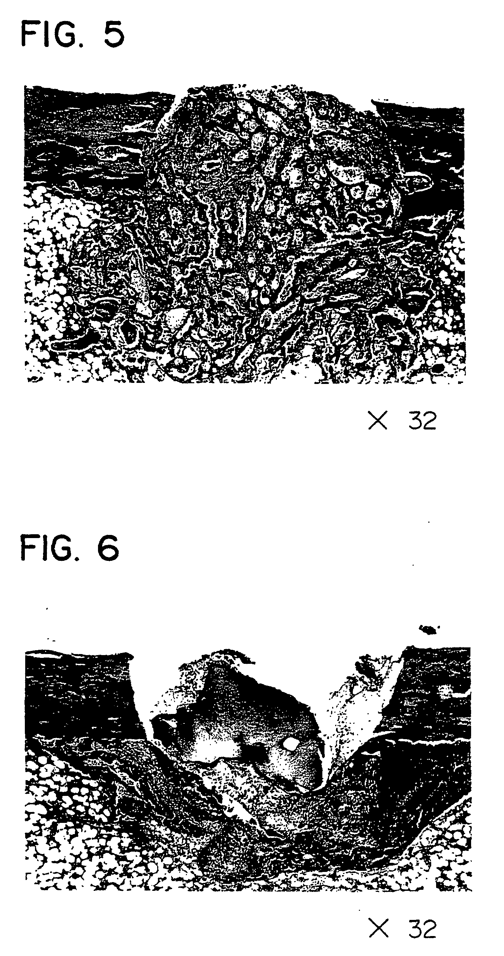

Comparing, for example, the microscopic photograph (of animal showing remarkable bone formation) in Group of Example 4 which is attached as Fig. 5 and the microscopic photograph (of animal showing moderate bone formation) in Group of Example 4 which is not attached with the microscopic photograph (of animal showing slight bone formation) in Control Group 4B which is attached as Fig. 6, slight new bones are merely noted at the bone defect site of the microscopic phctograph of Control Group 4B shown in Fig. 6, whereas large amounts of new bones are noted at the bone defect site of the microscopic photograph of Group of Example 4, shown in Fig. 5 and of the microscopic photograph of Group of Example 4 which is not attached. Further comparison between the microscopic photograph shown in Fig. 5 and the microscopic photograph (of animal showing remarkable bone formation) in Control Group 4A which is not attached reveals that Fig. 5 shows healing image almost equal to that of the microscopic photograph of Control Group 4A not attached. From these results, it is evident that the use of the present invention for the treatment of bone diseases possesses the excellent effect of accelerating bone formation.

### Example 5

In addition to human bFGF, the gel of human bFGF analogue (polypeptide shown by Sequence No. 3 in Table 3 attached in amino acid sequence of which 69 cysteine (Cys) and 87 cysteine (Cys) are substituted with serine (Ser), respectively) obtained from E. coli by genetic recombination technology was used. Volume, bone mineral content (BMC) and bone mineral density (BMD) of fractured callus were assessed in a similar manner to the case of Example 3.

These results (3 weeks after fracture) are presented in Table 5.

**Table 5**

| | Callus volume (n) (mm³) | BMC of callus (mg) | BMD of callus (mg/cm²) |
|---|---|---|---|
| Group A of Example 5 (50 µg of bFGF) | 20.2±7.2(6) | 7.1±0.6 | 9.8±2.1 |
| Group B of Example 5 (50 µg of bFGF anologue) | 22.3±6.1(5) | 6.9±1.1 | 9.1±2.8 |
| Control group A | 6.8±3.8(5) | 2.6±1.5 | 2.4±1.3 |
| Control group B | 10.4±4.5(5) | 2.9±1.7 | 4.1±2.1 |
| Non-diabetes control group | 8.8±2.2(5) | 4.2±2.6 | 5.8±4.0 |

As is clear from Table 5, volume, BMC and BMD of bFGF analogue group (Group B of Example 5) were as large as those of bFGF group (Group A of Example 5), indicating that human bFGF analogue has an equivalent effect to bFGF of the present invention.

### Toxicity

Six (6) male mice weighing 22 to 25 g were grouped in one group, and a plurality of groups were subjected to test on toxicity. Human bFGF solution (concentration of 3.3 mg/ml) equivalent to those used in Examples 1 through 4 was subcutaneously administered to each animal in a dose corresponding to the body weight. Based on mortality 72 hours after, LD₅₀ value was determined.

As the result, the LD₅₀ value of human bFGF was 75 mg/kg or more.

As stated above, according to the present invention, there is provided a novel use for the bFGF-analogues shown in claim 1 for the treatment of bone diseases which can shorten the healing period of various fractures including acceleration of bone formation for bone defect, and can improve reduced bone strength accompanied by various diseases.

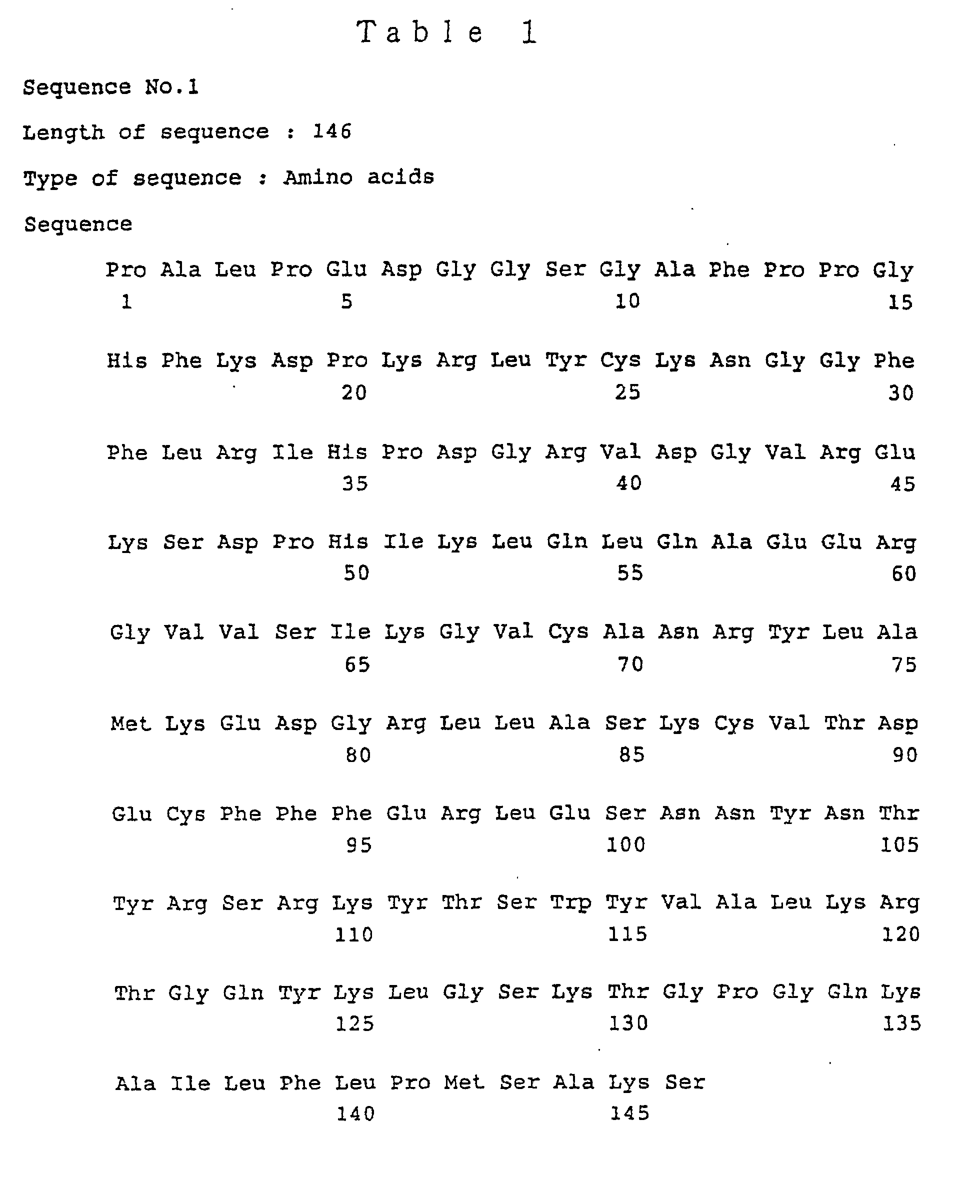

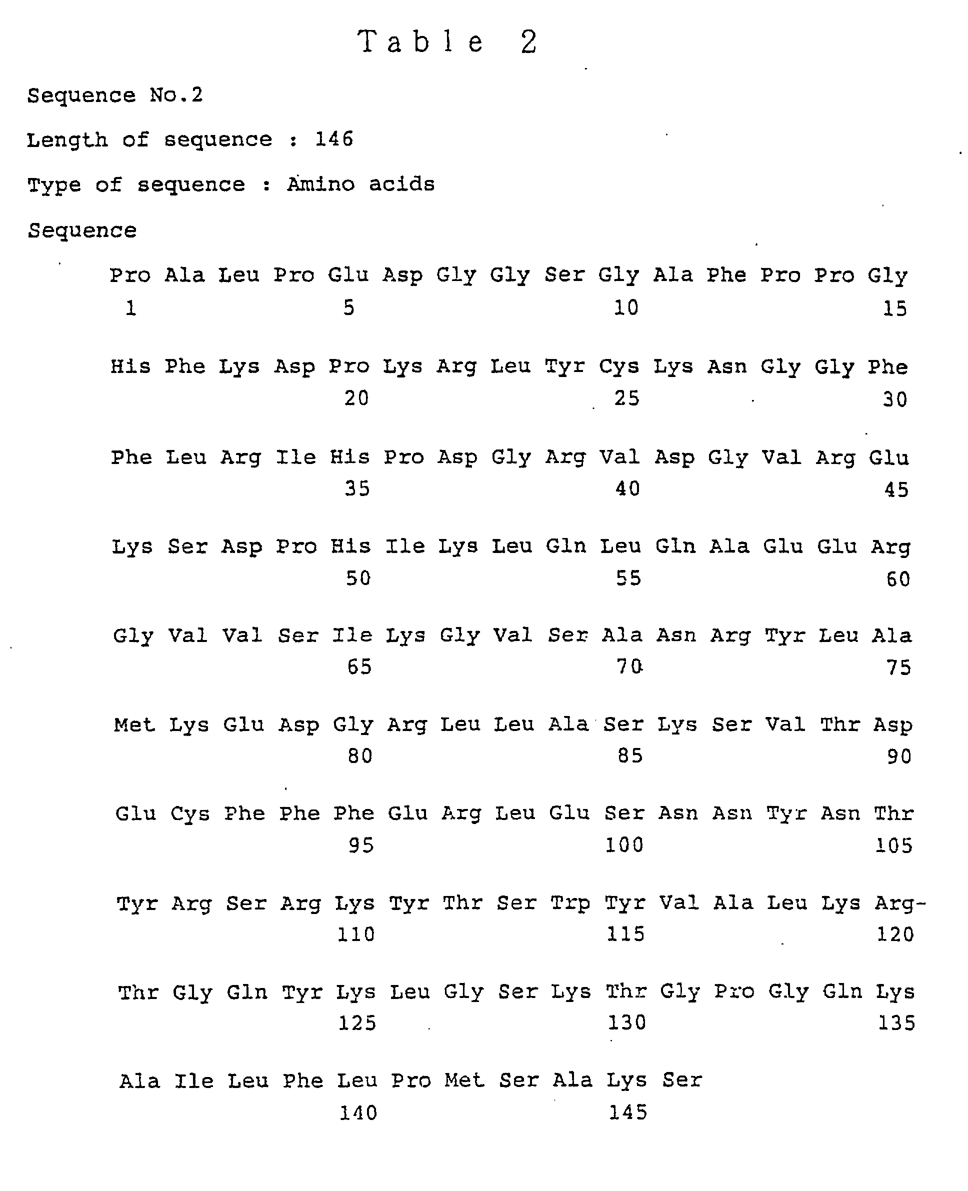

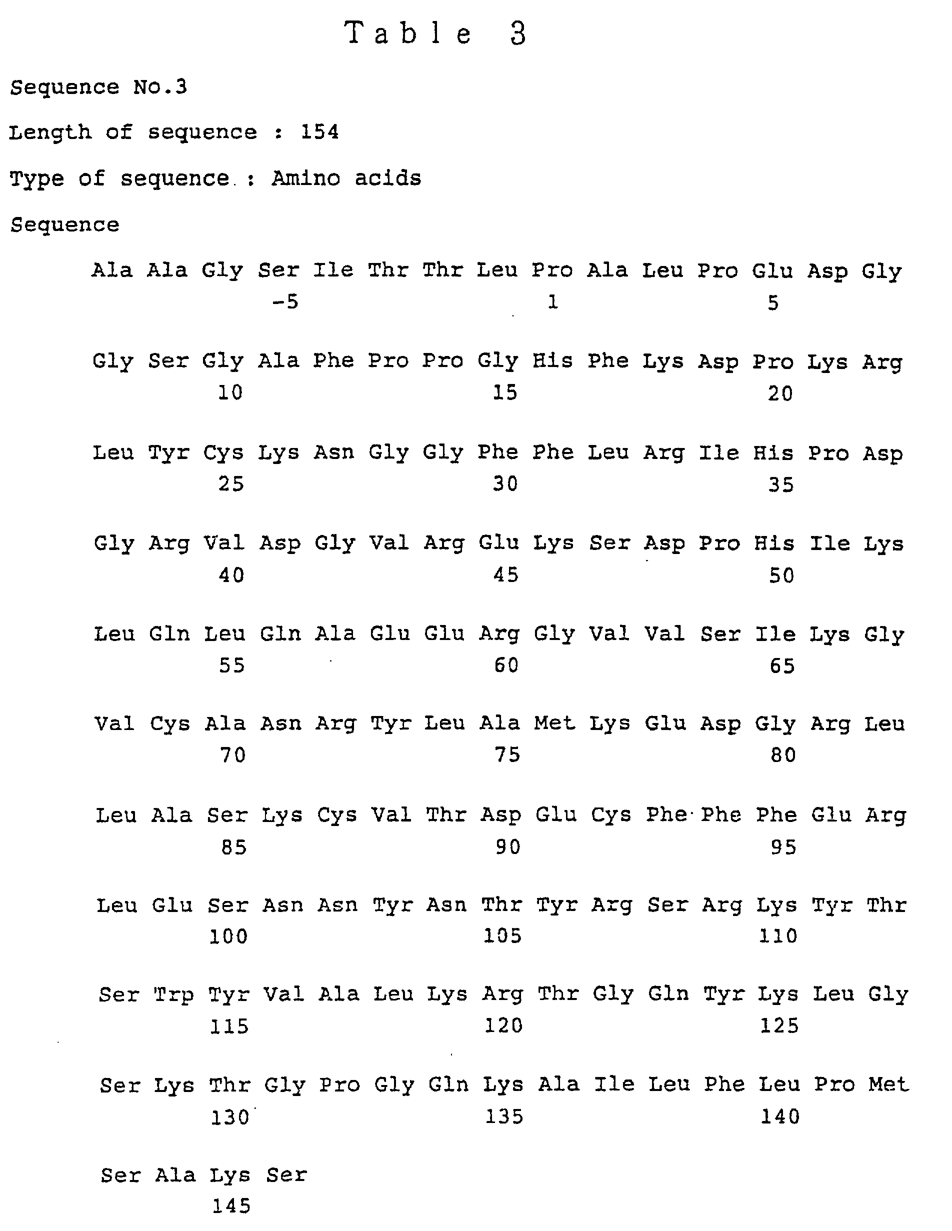

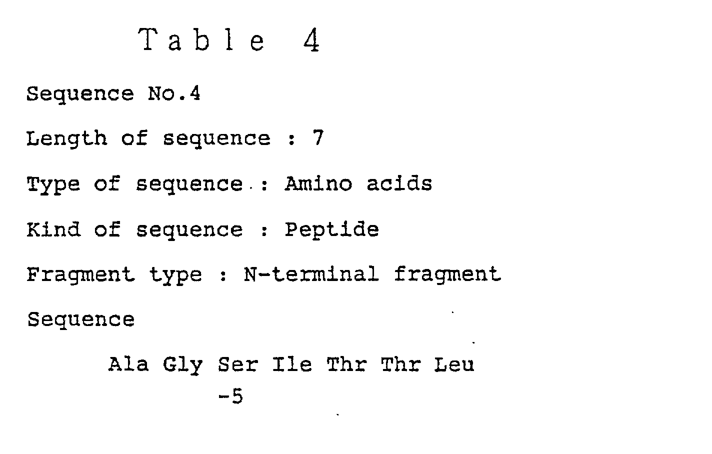

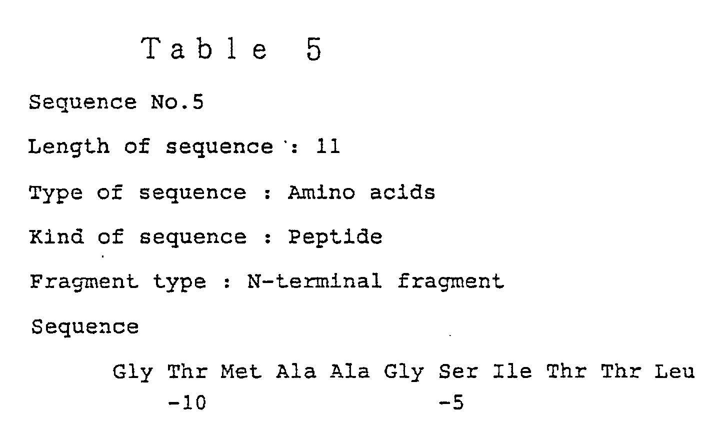

## Claims

1. Use of a human basic fibroblast growth factor (bFGF) analogue, which has the following amino acid sequence: 
Ala Ala Gly Ser Ile Thr Thr Leu Pro Ala Leu Pro Glu Asp Gly
-5 1 5
Gly Ser Gly Ala Phe Pro Pro Gly His Phe Lys Asp Pro Lys Arg
10 15 20
Leu Tyr Cys Lys Asn Gly Gly Phe Phe Leu Arg Ile His Pro Asp
25 30 35
Gly Arg Val Asp Gly Val Arg Glu Lys Ser Asp Pro His Ile Lys
40 45 50
Leu Gln Leu Gln Ala Glu Glu Arg Gly Val Val Ser Ile Lys Gly
55 60 65
Val Cys Ala Asn Arg Tyr Leu Ala Met Lys Glu Asp Gly Arg Leu
70 75 80
Leu Ala Ser Lys Cys Val Thr Asp Glu Cys Phe Phe Phe Glu Arg
85 90 95
Leu Glu Ser Asn Asn Tyr Asn Thr Tyr Arg Ser Arg Lys Tyr Thr
100 105 110
Ser Trp Tyr Val Ala Leu Lys Arg Thr Gly Gln Tyr Lys Leu Gly
115 120 125
Ser Lys Thr Gly Pro Gly Gln Lys Ala Ile Leu Phe Leu Pro Met
130 135 140
Ser Ala Lys Ser
145
 wherein said analogue is the one in which a segment composed of 8 amino acids having the following sequences: 
Ala Ala Gly Ser Ile Thr Thr Leu
-5 -1
 is added to a natural human basic fibroblast growth factor composed of 146 amino acids at the N terminus thereof, or the above human basic fibroblast growth factor analogue wherein the 69 cysteine (cys) and the 87 cysteine (cys) are each substituted with serine (Ser), for the preparation of a medicament for the treatment of traumatic bone fractures, fatigue bone fractures and pathologic bone fractures.

2. The use according to claim 1 for the preparation of a medicament for the treatment of a bone fracture patient having diabetes or a disease treated by steroid administration.

3. The use according to claim 1 for the preparation of a medicament for the treatment of a pathologic fracture accompanied by osteoporosis.

4. The use according to any of claims 1 to 3 for the preparation of a medicament for the acceleration of callus formation.

5. A use according to claim 1, wherein the medicament is in the form of a solution.

6. A use according to claim 5, wherein at least physiological saline and/or buffer is contained in the medicament in addition to said basic fibroblast growth factor analogue.

7. A use according to claim 1, wherein the medicament is in the form of a gel.

8. A use according to claim 1, which comprises applying the medicament for the treatment of the bone diseases to or around the affected site.

## Patentansprüche

1. Verwendung eines Analogons eines menschlichen Fibroblastwachstumsfaktors (bFGF), welches die folgende Aminosäurensequenz aufweist: 
Ala Ala Gly Ser Ile Thr Thr Leu Pro Ala Leu Pro Glu Asp Gly
          -5                  1                   5

Gly Ser Gly Ala Phe Pro Pro Gly His Phe Lys Asp Pro Lys Arg
        10                  15                  20

Leu Tyr Cys Lys Asn Gly Gly Phe Phe Leu Arg Ile His Pro Asp
        25                  30                  35

Gly Arg Val Asp Gly Val Arg Glu Lys Ser Asp Pro His Ile Lys
        40                  45                  50

Leu Gln Leu Gln Ala Glu Glu Arg Gly Val Val Ser Ile Lys Gly
        55                  60                  65

Val Cys Ala Asn Arg Tyr Leu Ala Met Lys Glu Asp Gly Arg Leu
        70                  75                  80

Leu Ala Ser Lys Cys Val Thr Asp Glu Cys Phe Phe Phe Glu Arg
        85                  90                  95

Leu Glu Ser Asn Asn Tyr Asn Thr Tyr Arg Ser Arg Lys Tyr Thr
        100                 105                 110

Ser Trp Tyr Val Ala Leu Lys Arg Thr Gly Gln Tyr Lys Leu Gly
        115                 120                 125

Ser Lys Thr Gly Pro Gly Gln Lys Ala Ile Leu Phe Leu Pro Met
        130                 135                 140

Ser Ala Lys Ser
        145
 wobei das Analogon dasjenige ist, in welchem ein aus 8 Aminosäuren bestehendes Segment mit den folgenden Sequenzen: 
Ala Ala Gly Ser Ile Thr Thr Leu
-5 -1
 einem natürlichen menschlichen Fibroblastwachstumsfaktor, der aus 146 Aminosäuren an seinem N-Ende besteht, oder dem oben genannten Analogon des menschlichen basischen Fibroblastwachstumsfaktors, in welchem das 69 Cystein (cys) und das 87 Cystein (cys) jeweils durch Serin (Ser) ersetzt sind, hinzugefügt wird, zur Herstellung eines Medikamentes zur Behandlung von traumatischen Knochenfrakturen, Verschleißknochenfrakturen und pathologischen Knochenfrakturen.

2. Verwendung gemäß Anspruch 1 zur Herstellung eines Medikamentes zur Behandlung einer Knochenfraktur bei einem Patienten, der an Diabetes oder einer mit Steroid-Verabreichung behandelten Krankheit leidet.

3. Verwendung gemäß Anspruch 1 zur Herstellung eines Medikamentes zur Behandlung einer pathologischen Fraktur, die mit Osteoporose einhergeht.

4. Verwendung gemäß irgendeinem der Ansprüche 1 bis 3 zur Herstellung eines Medikamentes zur Beschleunigung der Kallusbildung.

5. Verwendung gemäß Anspruch 1, wobei das Medikament in Form einer Lösung vorliegt.

6. Verwendung gemäß Anspruch 5, wobei wenigstens eine physiologische Kochsalzlösung und/oder ein Puffer in dem Medikament, zusätzlich zu dem basischen Fibroblastwachstumsfaktor-Analogon, enthalten ist.

7. Verwendung gemäß Anspruch 1, wobei das Medikament in Form eines Gels vorliegt.

8. Verwendung gemäß Anspruch 1, welche das Auftragen des Medikamentes zur Behandlung von Knochenkrankheiten auf oder um die betroffene Stelle herum beinhaltet.

## Revendications

1. Utilisation d'un analogue du facteur de croissance basique des fibroblastes (bFGF) humain, qui a la séquence d'aminoacides suivante: 
Ala Ala Gly Ser Ile Thr Thr Leu Pro Ala Leu Pro Glu Asp Gly
            -5                  1                   5
Gly Ser Gly Ala Phe Pro Pro Gly His Phe Lys Asp Pro Lys Arg
        10                  15                  20
Leu Tyr Cys Lys Asn Gly Gly Phe Phe Leu Arg Ile His Pro Asp
        25                  30                  35
Gly Arg Val Asp Gly Val Arg Glu Lys Ser Asp Pro His Ile Lys
        40                  45                  50
Leu Gln Leu Gln Ala Glu Glu Arg Gly Val Val Ser Ile Lys Gly
        55                  60                  65
Val Cys Ala Asn Arg Tyr Leu Ala Met Lys Glu Asp Gly Arg Leu
        70                  75                  80
Leu Ala Ser Lys Cys Val Thr Asp Glu Cys Phe Phe Phe Glu Arg
        85                  90                  95
Leu Glu Ser Asn Asn Tyr Asn Thr Tyr Arg Ser Arg Lys Tyr Thr
        100                 105                 110
Ser Trp Tyr Val Ala Leu Lys Arg Thr Gly Gln Tyr Lys Leu Gly
        115                 120                 125
Ser Lys Thr Gly Pro Gly Gln Lys Ala Ile Leu Phe Leu Pro Met
        130                 135                 140
Ser Ala Lys Ser
        145
 ledit analogue étant celui dans lequel un segment composé de 8 aminoacides, ayant la séquence suivante: 
Ala Ala Gly Ser Ile Thr Thr Leu
-5 -1
 est ajouté à l'extrémité N-terminale du facteur de croissance basique des fibroblastes humain composé de 146 aminoacides, ou de l'analogue du facteur de croissance basique des fibroblastes humain ci-dessus dans lequel la cystéine (Cys) 69 et la cystéine (Cys) 87 sont remplacées chacune par une sérine (Ser), pour la préparation d'un médicament destiné au traitement de fractures osseuses traumatiques, de fractures osseuses de fatigue et de fractures osseuses pathologiques.

2. Utilisation selon la revendication 1, pour la préparation d'un médicament destiné au traitement d'un patient souffrant d'une facture osseuse et ayant un diabète ou une maladie traitée par l'administration de stéroïdes.

3. Utilisation selon la revendication 1 pour la préparation d'un médicament destiné au traitement d'une fracture pathologique accompagnée d'ostéoporose.

4. Utilisation selon l'une quelconque des revendications 1 à 3 pour la préparation d'un médicament destiné à accélérer la formation de cals.

5. Utilisation selon la revendication 1, dans laquelle le médicament est sous forme d'une solution.

6. Utilisation selon la revendication 5, dans laquelle le médicament contient au moins du sérum physiologique et/ou un tampon en plus dudit analogue de facteur de croissance basique des fibroblastes.

7. Utilisation selon la revendication 1, dans laquelle le médicament est sous forme d'un gel.

8. Utilisation selon la revendication 1, qui comprend l'application du médicament destiné au traitement des maladies osseuses sur le site de l'affection ou autour du site de l'affection.
